# EUROPEAN PATENT APPLICATION

(11) **EP 4 520 253 A1**
(43) Date of publication of application: **12.03.2025**
(21) Application number: 23315342.8
(22) Date of filing: 06.09.2023
(51) Int. Cl.: A61B 3/103, A61B 3/028

(54) **RETINOSCOPIC AID DEVICE**

(71) Applicant: Essilor International, 94220 Charenton-Le-Pont (FR)
(72) Inventor: Grand-Clement, Didier, 93100 MONTREUIL (FR)
(74) Representative: Innovincia

(57) **Abstract**

The invention relates to a retinoscopic aid device (3) for use with a portable streak retinoscope (17) and with an automated phoropter (1) for testing an individual's eye comprising :
- a base plate (11) configured to be detachable fastened to the automated phoropter (1),
- an optical display unit (13) configured to display an orientable straight line mark (21), the optical display unit (13) being supported by the base plate (11),
- a control unit (3B) of the optical display unit (13) which is configured to orient the straight line mark (21) of the optical display unit (13) according to a control command of a user allowing the user (U) to orient the straight line mark (21) according the streak orientation of the portable streak retinoscope when looking through the portable retinoscope (17).

## Description

### TECHNICAL FIELD

The present disclosure relates to a retinoscopic aid device for use with a portable streak retinoscope and with an automated phoropter for testing an individual's eye.

### BACKGROUND OF THE DISCLOSURE

Retinoscopy is a standard practice for objective evaluation of patient refraction and part of standard academic cursus in optometry. It requires a retinoscope and a set of trial lenses for correcting refraction error or can be done in a phoropter. Such phoropter may be of a classical kind with a set of lenses of different powers that can be successively disposed along the optical paths reaching the eyes of the subject, to change the vision correction powers provided to each eye. The set of lenses is manually operated by the practitioner or user from the front face of the phoropter (opposed to the subject) to change of lenses disposed along the optical path of the subject's eyes and this front face also supports visual axis references able to surround the lenses disposed along the optical path.

It is particularly suitable for kids or disabled patients unable to perform standard subjective refraction.

Current refraction techniques use the streak retinoscope for measuring optical errors. After the practitioner has measured the patient's optical error by changing the mechanical phoropter or trial frame lenses, the values of those lenses are consulted to determine the patient's ocular correction. These refraction techniques essentially use the phoropter or trial frame lenses to make the light rays emanating from the retinoscope conjugate to the patient's fundus. It is generally a four steps method which is for example described in Retinoscopy 101 - American Academy of Ophthalmology (aao.org)".

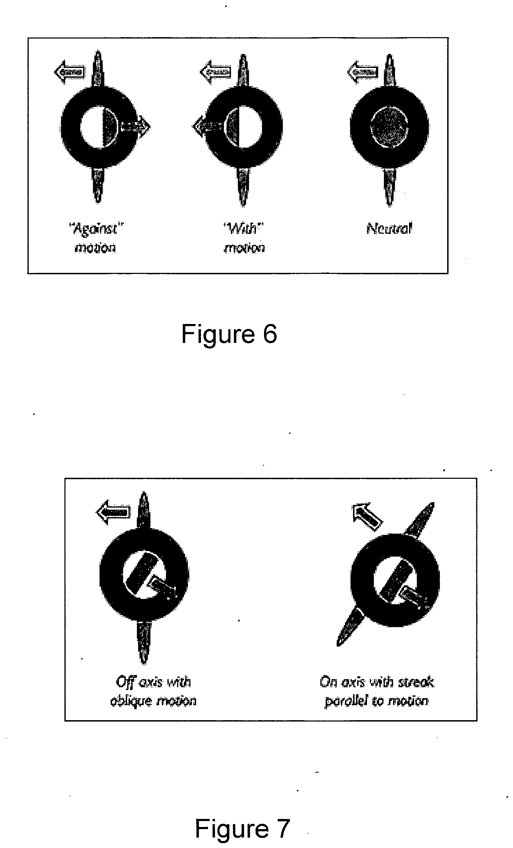

More precisely, when the practitioner or user implements a retinoscopy with such phoropter, the practitioner illuminates the subjects'eye with the retinoscope's streak through the current lens disposed along the optical path, moves this streak side to side, and observes a reflex motion or light reflex in the patient's pupil through the retinoscope to determine if the light reflex in the patient's pupil moves "with" or "against" motion of the retinoscope's streak (see figure 6).

The examination takes place as follows for a subject having only spherical refractive error:
- If the light reflex moves "with" (same way) the retinoscope's streak movement, then the practitioner needs to add increasing positive power selecting with new lens(es), until neutrality (bright red reflex without any motion that fills the pupil) is achieved.
- On the contrary, if the light reflex moves "against" (opposite way) the retinoscope's streak movement, then the practitioner needs to add increasing negative power(s) with new lens(es) until neutrality is achieved.
- It is a good practice to always start from a "move with" setting of the phoropter's lens .

For a subject having cylindrical refractive error (see figure 7), as the practitioner or user moves the retinoscope's streak side to side, the light reflex in the patient's pupil moves obliquely parallel to a specific direction that allows the determination of the axis of the subject's astigmatism. As explained above, this motion can be neutralized with the selection of lenses of different power. Moreover, once a direction has been neutralized, the Practitioner can use the visual axis references on the front face of the phoropter to quantify the orientation of the specific direction and deduce the astigmatism axis.

Currently in the field of ophthalmology and optometry, automated systems are often used to control motorized phoropters, chart projectors, and other equipment providing increased efficiency in the exam, including the capture and transfer of data to electronic medical records. Motorized phoropters usually have set of lenses with discrete power disposed along the optical paths reaching the eyes of the subject to change the vision correction powers provided to each eye by the change in the set of lenses. The change of the powers of a motorized phoropter, and the subjective tests displayed by the screen can be controlled automatically by a computer (keyboard) with the practitioner sitting aside or even being remote and not manually by the practitioner or user with the practitioner in front of the phoropter. Therefore, such motorized phoropters are usually deprived from any axis indication.

The situation is similar for improved phoropter which usually have tunable lenses of continuous variable power disposed along the optical paths reaching the eyes of the subject to change the vision correction powers provided to each eye by the change of the power of the tunable lens.

Therefore automated (i.e. motorized or improved) phoropters are not suited to perform retinoscopy because of lack of axis indication on the phoropter head when determining axis, difficulties in driving the improved phoropter while keeping arm's distance and difficulty to evaluate distance between the patient and the retinoscope.

The present invention proposed to overcome at least partially this drawback of automated phoropters.

### SUMMARY OF THE DISCLOSURE

In order to achieve this goal, the present disclosure proposes a retinoscopic aid device for use with a portable streak retinoscope and with an automated phoropter for testing an individual's eye comprising
- a base plate configured to be detachable fastened to the automated phoropter,
- an optical display unit configured to display an orientable straight line mark, the optical display unit being supported by the base plate,
- a control unit of the optical display unit which is configured to orient the straight line mark of the optical display unit according to a control command of a user allowing the user to orient the straight line mark according the streak orientation of the portable streak retinoscope when looking through the portable retinoscope.

According to further aspects taken alone or in combination relating to the above defined retinoscopic aid device :
The base plate and the optical display unit can present a central opening of the size of an observation window of the automated phoropter allowing them to be centered around the observation window and observation of a patient's eye through the central opening and observation window.

The optical display unit may comprise a ring of light sources, in particular LEDs, which are regularly disposed in a circle arrangement around a center, and the control unit can be configured to switch the light sources according to an illumination pattern representing the straight line mark.

The control unit is for example configured such that only two light sources which are opposed with regard to the center may be switched on at the same time, these two switched on light sources representing the straight line mark.

The control unit of the optical display unit may be manually operable.

The control unit of the optical display unit comprises for example a scroll wheel unit disposed at distance to the display unit.

The control unit of the optical display unit can also be integrated to the automated phoropter and may be configured to be controlled by a user interface of the automated phoropter.

The control unit of the optical display unit can be integrated to the automated phoropter and may be configured to control functions of the automated phoropter in particular variation of lens power along an optical path.

The retinoscopic aid device can further comprise a bearing arranged between the base plate and the optical display unit, the optical display unit having two light sources which are disposed in opposition with regard to the rotation center and represent the straight line mark.

The retinoscopic aid device may further comprise a display screen, in particular an LCD screen for display of a value of the observed streak orientation and/or of a value of a power of a lens of the automated phoropter.

According to one aspect, the at least one detachable fastener comprises for example a magnet.

According to another aspect, the at least one detachable fastener comprises for example a scratch fastener.

The invention also relates to a system for optimization of human visual function comprising an automated phoropter and at least one retinoscopic aid device as defined above.

With regard to the system, the base plate and the optical display unit of the retinoscopic aid device presents for example a central opening of the size of an observation window of the automated phoropter allowing the retinoscopic aid device to be centered with regard to the observation window and observation of a patient's eye through the central opening and observation window.

The invention further relates to a method for testing an individual's eye with use of a portable streak retinoscope and an automated phoropter equipped with an retinoscopic aid device as defined above, having at least the following steps :
- looking through the streak retinoscope in direction of an individual's eye, the individual being looking through the automated phoropter ,
- adjust the straight line mark of the optical display unit to be orientated according the streak image orientation of the retinoscope,
- adjust cylindrical power of the automated phoropter according the determined straight line mark (21) orientation of the optical display unit (13).

### BRIEF DESCRIPTION OF THE DRAWINGS

Other advantages and features will become apparent upon reading the description of the following figures, among which:
- Figure 1 shows a schematically perspective view of an automated phoropter equipped with a retinoscopic aid device according to an exemplary embodiment of the invention,
- Figure 2 shows a top view of an example of an optical display unit of the retinoscopic aid device of figure 1,
- Figure 3 shows a perspective view of the optical display unit of figure 2,
- Figure 4 shows simplified side view a practitioner and a patient using the retinoscopic aid device according to the invention,
- Figure 5 shows a top view of an example of an optical display unit with two light sources activated for explaining the functioning of the retinoscopic aid device
- Figure 6 shows three pictures in order to explain retinoscopy examination for a subject having only spherical refractive error, and
- Figure 7 shows two pictures in order to explain retinoscopy examination in a case of a subject having cylindrical refractive error.

### DETAILED DESCRIPTION

On all the figures, the same elements bear the same reference numbers.

The following embodiments are only examples. Although the description refers to one or several embodiments, the invention is not limited to these embodiments. In addition, a feature described in relationship with one embodiment may also concern another embodiment even if this is not mentioned expressively. Simple features of different embodiments may also be combined to provide further realizations.

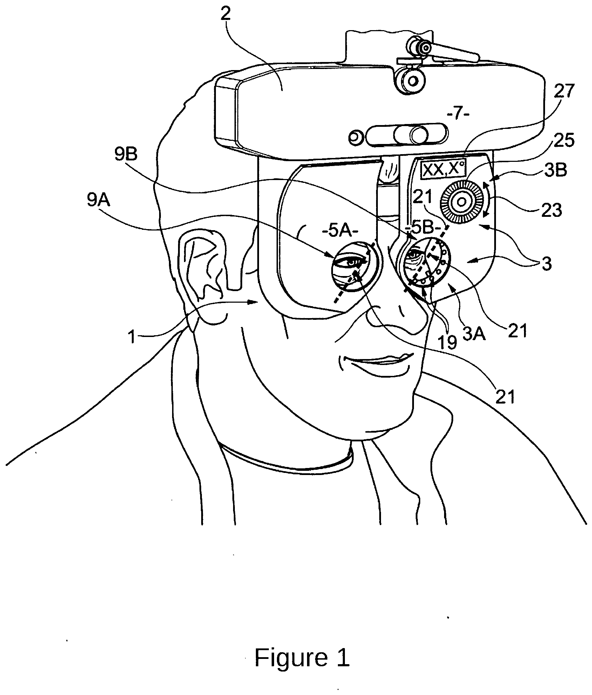

Figure 1 shows a schematically perspective view of a part of an automated phoropter 1 which is the refraction head 2, equipped with a retinoscopic aid device 3 according to an exemplary embodiment of the invention.

Such kind of phoropter and display unit used in the optometry device according to the invention may be those of the device described in document EP3128894 or US20040032568 or is for example a model Vision R 800 manufactured by the applicant described in the above mentioned user manual (https://www.essilorinstrumentsusa.com/wp-content/uploads/2019/01/Vision-R-800-User-Manual-US.pdf).

There exist also some compact automated refraction device including in a casing, an automatic phoropter (either motorized or improved), a display element such as a screen, and an optical system allowing to change the virtual distance of observation of the screen by the subject from near vision distance (for example 40 cm) to far vision distance (for example 5-6 meters or infinity). Some of this compact device can offer direct view of the phoropter head or can be equipped with a monitoring window (not illustrated) located on the casing and allowing the practitioner or user to observe the pupils of the subject looking through the phoropter's lens. Such compact version also be improved by the features of the here disclosed invention.

In figure 1, the refraction head 2 of the automated phoropter is represented from the user or practitioner side and the patient side where the individual is sitting for examination of his eyes is on the opposite side, behind the refraction head 2 as shown in figure 1.

The refraction head 2 comprises two halves 5A and 5B which are arranged in a sliding manner and disposed on a cross bar 7. Each half 5A or 5B comprises an observation window 9A or 9B through which the patient / individual will look with his eyes through the phoropter's lens being either the discrete lens that was automatically put on the optical path by the control unit of a motorized phoropter, or a tunable lens whose power is automatically set to a specific value by the control unit of an improved phoropter. The halves 5A and 5B are generally surrounded by a casing made of plastic with individual attached magnets or into the plastic embedded / integrated magnetic particles for attachment to halves 5A or 5B, or by a metallic casing, for example a ferromagnetic casing.

The halves 5A and 5B can be displaced in horizontal position, parallel to the cross bar 7 in order to adjust their distance with respect to each other in a way that the patient / individual can look at the same time through each of the observation windows 9A and 9B.

As shown in figure 1, right half 5B is equipped with a detachable retinoscopic aid device 3 allowing to facilitate to determine cylindrical power and streak axis determination by retinoscopy in case of astigmatism.

In figure 1, one can see two parts 3A and 3B of the retinoscopic aid device 3.

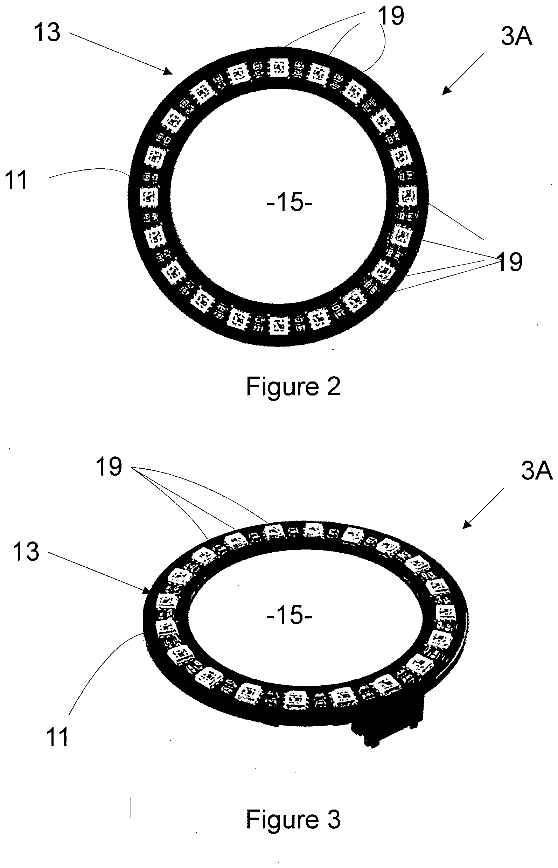

Part 3A is shown in more detail in figures 2 and 3 and comprises a base plate 11 and an optical display unit 13 supported by the base plate 11.

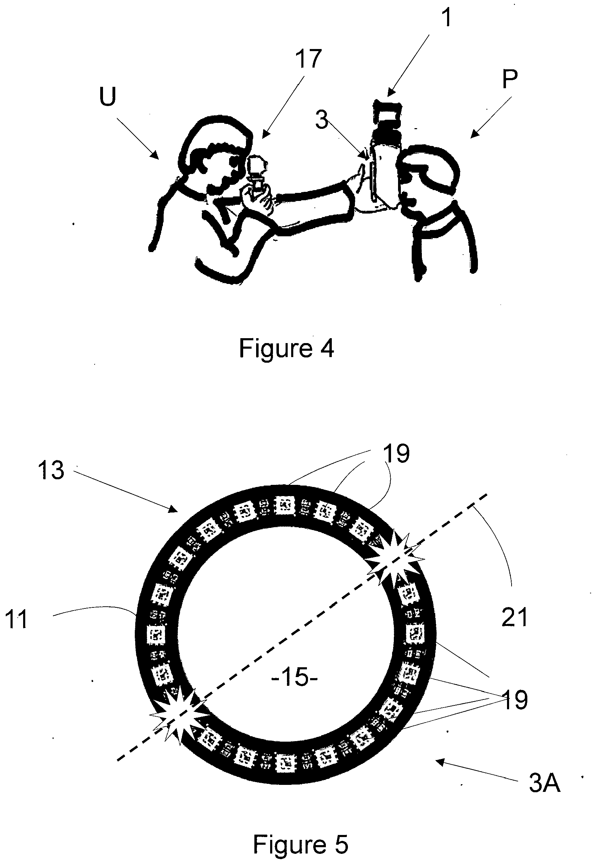

The base plate 11 and the optical display unit 13 present a central opening 15 of the size of an observation window 9A, 9B of the automated phoropter 1 allowing part 3A formed of the base plate 11 and the optical display unit 13 of the retinoscopic aid device 3 to be centered around the observation window 9A or 9B and allowing observation of a patient's eye through the central opening 15 and observation window 9A, 9B while manual examination with a streak retinoscope 17 (see figure 4).

As shown in figures 2 and 3, the optical display unit 13 comprises a ring of light sources 19 (only some of them are exemplary referenced in the drawing), in particular LEDs which may be powered for example by a battery and which are regular disposed in a circle arrangement around a center. The number of light sources 19 depends on the angular resolution which is desired to be achieved. As an example in figures 2, 3, and 5, the optical display unit comprises 20 light sources allowing to reach an angular resolution of 18°. In case for example an angular resolution of 5° shall be reached, the number of light sources 19 needs to be 72. As will be explained later, the optical display unit 13 is configured to display an orientable straight line mark 21 (shown in figure 5).

The base plate 11 is configured to be detachably fastened to the automated phoropter 1, in particular at the observation window 9A or 9B, on the practitioner or user side.

According to a specific embodiment the base plate 11 can have an extended shape corresponding to the shape of the casing and cover of the phoropter's halves 5A or 5B. In this case, one might replace the original casing of the halves 5A /5B. In other examples, with extended shape, the base plate 11 can partially cover halves 5A or 5B.

Several solutions may be envisaged among which a "magnetic solution" where the base plate 11 is for example made of a magnetic material and can be attached directly to the ferromagnetic part of the casing of the automated phoropter 1.

Another solution may comprise individual magnets which are for example fixed in particular by gluing to the back side of the base plate 11 which is opposite to the side supporting the light sources 19 and whose locations corresponds to metallic parts in casing of halves 5A or 5B,

In case the phoropter's casing is not metallic but for example plastic, one might foresee a solution with scratch fasteners where one element of the scratch fastener is for example fixed by gluing on the automated phoropter 1, in particular in form of a ring around the observation windows 9A and 9B and the other on the backside of the base plate 11.

Going back to figure 1, the retinoscopic aid device 1 comprises a part 3B which is a control unit configured to switch the light sources 19 according to an illumination pattern representing a virtual straight line mark 21.

This is for example shown in figure 5 where only two light sources 19 which are located on opposite sides of the ring of light sources 19, are illuminated. The straight line 21 that is a virtual line, is shown on figure 5 for explanation but in normal use, the practitioner or user needs to image the straight line 21 by connecting in his mind the illuminated light sources 19 by a line, The control unit 3B is configured such that only two light sources 19 which are opposed with regard to the center may be switched on at the same time, these two switched on light sources 19 representing two points belonging to the straight line mark 21.

The control unit which is also referenced 3B comprises for example a scroll wheel unit 25 as represented in the figure 1 or a sliding bar, and is disposed at distance to the part 3A of the detachable retinoscopic aid device 3. The control unit 3B is manually operable, for example with a thumb or the index finger tip. The control unit 3B is also detachably fixed to the automated phoropter 1. Similar as explained with regard to the base plate 11, the control unit 3B may be removably attached by magnets or scratch fasteners.

In case the base plate 11 has an extended shape corresponding to the shape of the casing and cover of halves 5A or 5B as described above, the control unit 3B can be integrated / supported by the base plate 11 of extended shape.

The scroll wheel unit 25 can rotate as shown by arrow 23 in figure 1. The display unit 13 and the control unit 3B are for example equipped with wireless communications means based for instance on well known communication protocols like Bluetooth or Zigbee (registered trademarks) or with wired communication when located on the same platform, potentially connected with the Keyboard. As both units 3B and 13 are quite close to another and in general only a one direction communication is required to transmit control commands, one might also use for example other or simpler NFC communication protocols or data exchanges based on active or passive RFID technology.

In a more sophisticated configuration the control unit 3B can both interact with part 3A and the phoropter's control unit, in particular in allowing control of some functions which are normally controlled by the operator via keyboard control, in particular to control variation of lens power in the case of an improved phoropter, or the automatic change of the lens (and therefore the power of the lens) to be put on the optical path in the case of a motorized phoropter. In this case, one might envisage that the scroll wheel unit 25 comprises in addition a central push button which may allow the operator to change for example between a first mode of control of part 3A in particular the display unit 13 and a second mode of control of lens power of the lens along the oprical path of the phoropter 1.

Thus in function of the angular position of the scroll wheel unit 25, two opposite specific light sources 19 will be illuminated or activated in order to display straight line mark 21 which orientation for example with regard to a horizontal line depends on the angular position of the scroll wheel unit 25. The practitioner or user will actuate the scroll wheel unit 25 to align the straight line mark 21 with the axis of the streak orientation of the light reflex in the patient's pupil as observed in the retinoscope, allowing to determine the axis of the subject's astigmatism.

The fact that the control unit 3B is detachably fixed to the automated phoropter 1 allows to ease the retinoscopic examination because as shown in figure 4, the practitioner or user U can hold in one hand the retinoscope 17 and can control at arm length the control unit 3B.

According to a not shown embodiment, 7 the control unit 3B of the optical display unit 11 may be integrated to the automated phoropter 1 and is for example configured to be controlled by a user interface in particular a keyboard unit of the automated / motorized phoropter 1.
As shown in figure 1, the retinoscopic aid device may comprise in a more sophisticated version a display screen 27, in particular an LCD screen which can communicate with the control unit 3B and displays a value for example of the orientation of the straight line mark 21 with respect to a straight vertical line in ° like shown "XX,X°". The display screen 27 may be detachable be fixed like the base plate 11 or the control unit 3B to the automated phoropter 1.

In case the base plate 11 has an extended shape corresponding to the shape of the casing and cover of halves 5A or 5B as described above, the display screen 27 can be integrated / supported by the base plate 11 of extended shape.

In case of the above described more sophisticated embodiment allowing with a central push button mode control selection, the display screen can also be involved and shows in the first mode described above the axis orientation optionally together with the current power of the lens along the optical path, and in the second mode of control the selected lens power.

A further embodiment not shown, the optical display unit 13 has two light sources which are disposed in opposition with regard to the rotation center and represent the straight line mark and the retinoscopic aid device 3 comprises a bearing arranged between the base plate 11 and the optical display unit 13 which plays at the same time the function of the control unit 3B for changing the orientation of the straight line mark 21.

Indeed, with regard to this example, when the two light sources are activated, the user can rotate the optical display unit 13 by taking it between the thumb and index finger tip during the retinoscopic examination in order to adapt the orientation of the straight line mark according to his observations.

Such measured orientation may be then transferred either automatically or manually to the phoropter keyboard for good orientation of the cylinder before astigmatic neutralization.

In figure 4 is shown a practitioner or user U who has in his one hand a retinoscope 17. The practitioner U is positioned at arm length from the automated phoropter 1. A patient or individual P is looking in direction to the practitioner U through the observation windows 9A and 9B (see figure 1) of the automated phoropter 1. With his other hand which is positioned on the control unit 3B, in particular the scroll wheel unit 25, the practitioner U can orient the straight line mark 21 (see on figure 5) according to his observations. By scrolling the scroll wheel unit 25, a control command is send to the display unit 13 and in function of the angular position of the scroll wheel, the light sources 19 are activated and representing the angular position of the scroll wheel and in particular the streak image orientation observed by the practitioner U through the retinoscope 17.

The angular position is then used to orient the phoropter cylinder, either manually on the keyboard after reading the value on display screen 27, or automatically through communication with the control unit of the phoropter 1.

Thus generally speaking, the control unit 3B of the optical display unit 13 is configured to orient the straight line mark 21 of the optical display unit 13 according to a control command of the practitioner U allowing him to orient the straight line mark 21 according to the observation of the streak orientation of the portable streak retinoscope 17 when looking through the portable retinoscope 17.

Thus, when using an automated phoropter 1, the retinoscopic examination of a patient P is eased and can be assured by practitioner in the following way.

The practitioner P looks through the streak retinoscope 17 in direction of one individual's eye, the individual or patient P being looking through the automated phoropter 1, in particular through the observation windows 9A/ 9B.

After neutralization of the first meridian, the practitioner P then adjusts according to his observations the straight line mark 21 of the optical display unit 13 to be orientated according the streak image orientation of the retinoscope 1 which he/she observes after 90° rotation.

This allows him/her then to adjust the axis of the cylindrical power of the automated phoropter 1 according to the determined straight line mark orientation of the optical display unit 13 and changing the cylindrical power of the variable cylinder along this axis to reach neutralisation of the second meridian.

Once he/she has terminated the examination of one eye of the patient P, he can make the same examination for the other eye. In order to do this, he can simply detach the optical display unit 13 from one observation window 9A for example and fixes it to the other observation window 9B of the automated phoropter 1. As an alternative, the phoropter can be equipped with two optical display units 13 for the two observation windows 9A, 9B according to the invention.

## Claims

1. Retinoscopic aid device (3) for use with a portable streak retinoscope (17) and with an automated phoropter (1) for testing an individual's eye comprising
- a base plate (11) configured to be detachable fastened to the automated phoropter (1),
- an optical display unit (13) configured to display an orientable straight line mark (21), the optical display unit (13) being supported by the base plate (11),
- a control unit (3B) of the optical display unit (13) which is configured to orient the straight line mark (21) of the optical display unit (13) according to a control command of a user allowing the user (U) to orient the straight line mark (21) according the streak orientation of the portable streak retinoscope when looking through the portable retinoscope (17).

2. Retinoscopic aid device (3) according to claim 1, where the base plate (11) and the optical display unit (13) present a central opening (15) of the size of an observation window (9A, 9B) of the automated phoropter (1) allowing them to be centered around the observation window (9A, 9B) and observation of a patient's eye through the central opening and observation window (9A, 9B).

3. Retinoscopic aid device (3) according to claim 1 or 2, where the optical display unit comprises a ring of light sources (19), in particular LEDs, which are regularly disposed in a circle arrangement around a center, and the control unit (3B) is configured to switch the light sources according to an illumination pattern representing the straight line mark (21).

4. Retinoscopic aid device according to claim 3, where the control unit (3B) is configured such that only two light sources (19) which are opposed with regard to the center may be switched on at the same time, these two switched on light sources (19) representing the straight line mark (21).

5. Retinoscopic aid device according to claim 3 or 4, where the control unit (3B) of the optical display unit (13) is manually operable.

6. Retinoscopic aid device according to claim 5, where the control unit (3B) of the optical display unit (13) comprises a scroll wheel unit (25) disposed at distance to the display unit (13).

7. Retinoscopic aid device according to claim 5, where the control unit (3B) of the optical display unit (13) is integrated to the automated phoropter (1) and is configured to be controlled by a user interface of the automated phoropter (1).

8. Retinoscopic aid device according to claim 5, where the control unit (3B) of the optical display unit (13) is integrated to the automated phoropter (1) and is configured to control functions of the automated phoropter (1) in particular variation of lens power along an optical path.

9. Retinoscopic aid device according to claim 1, further comprising a bearing arranged between the base plate (11) and the optical display unit (13), the optical display unit (13) having two light sources (19) which are disposed in opposition with regard to the rotation center and represent the straight line mark (21).

10. Retinoscopic aid device according to any of claims 1 - 9, further comprising a display screen (27), in particular an LCD screen for display of a value of the observed streak orientation and/or of a value of a power of a lens of the automated phoropter.

11. Retinoscopic aid device according to any precedent claim, where the at least one detachable fastener comprises a magnet.

12. Retinoscopic aid device according to any of claims 1- 10, where the at least one detachable fastener comprises a scratch fastener.

13. System for optimization of human visual function comprising an automated phoropter (1) and at least one retinoscopic aid device (3) according to any of claims 1-12.

14. System according to claim 13, where the base plate (11) and the optical display unit (13) of the retinoscopic aid device (3) presents a central opening of the size of an observation window (9A, 9B) of the automated phoropter (1) allowing the retinoscopic aid device (3) to be centered with regard to the observation window and observation of a patient's eye through the central opening and observation window (9A, 9B).

15. Method for testing an individual's eye with use of a portable streak retinoscope (17) and an automated phoropter (1) equipped with an retinoscopic aid device (3) according to any of claims 1 to 10, having at least the following steps :
- looking through the streak retinoscope (17) in direction of an individual's eye, the individual being looking through the automated phoropter (1),
- adjust the straight line mark (21) of the optical display unit (13) to be orientated according the streak image orientation of the retinoscope (17),
- adjust cylindrical power of the automated phoropter (1) according the determined straight line mark (21) orientation of the optical display unit (13).
